(19) Europäisches Patentamt
European Patent Office
Office européen des brevets

(11) Publication number: **0 266 549 B1**

(12) **EUROPEAN PATENT SPECIFICATION**

(45) Date of publication of patent specification: **19.06.91**

(51) Int. Cl.5: **C07C 215/62**, C07C 217/84, C07C 229/34, C07C 255/46, C07D 295/08, C07D 295/12, C07D 295/14, A61K 31/00

(21) Application number: **87114175.0**

(22) Date of filing: **29.09.87**

# (54) Cinnamyl amines, process for their preparation and pharmaceutical compositions containing them.

(30) Priority: **30.09.86 IT 2185786**

(43) Date of publication of application:
**11.05.88 Bulletin 88/19**

(45) Publication of the grant of the patent:
**19.06.91 Bulletin 91/25**

(84) Designated Contracting States:
**AT BE CH DE ES FR GB GR IT LI LU NL SE**

(56) References cited:
**EP-A- 0 187 639**
**US-A- 2 831 864**

**CHEMICAL ABSTRACTS, vol. 77, no. 21, 20th November 1972, page 402, abstract no. 139538q, Columbus, Ohio, US; H. BOEHME et al.: "Alpha-halo amines. 39. Reaction of substituted cinnamic acids, esters, and aldehydes with alpha-halo amines"**

(73) Proprietor: **BOEHRINGER MANNHEIM ITALIA S.P.A.**
**Via S. Uguzzone, 5**
**I-20126 Milan(IT)**

(72) Inventor: **Menta, Ernesto**
**Via Sant'Uguzzone, 5**
**I-20126 Milan(IT)**
Inventor: **Spinelli, Silvano**
**Via Sant'Uguzzone, 5**
**I-20126 Milan(IT)**
Inventor: **Gandolfi, Carmelo A.**
**Via Sant'Uguzzone, 5**
**I-20126 Milan(IT)**
Inventor: **Frigerio, Marco**
**Via Sant'Uguzzone, 5**
**I-20126 Milan(IT)**
Inventor: **Tofanetti, Odoardo**
**Via Sant'Uguzzone, 5**
**I-20126 Milan(IT)**
Inventor: **Tognella, Sergio**
**Via Sant'Uguzzone, 5**
**I-20126 Milan(IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**EP 0 266 549 B1**

**ARZNEIMITTEL FORSCHUNG, vol. 33, no. 8, 1983, pages 1142-1151, Aulendorf, DE; W. MEULDERMANS et al.: "Excretion and metabolism of flunarizine in rats and dogs"**

(74) Representative: **Weber, Manfred, Dr. et al**
**Boehringer Mannheim GmbH Patentabteilung Sandhofer Strasse 116**
**W-6800 Mannheim 31(DE)**

## Description

The present invention relates to cinnamylamines, hydroxy or alkylamino substituted, a method for their preparation and pharmaceutical and veterinary compositions containing them.

More particularly, the invention concerns compounds of general formula I

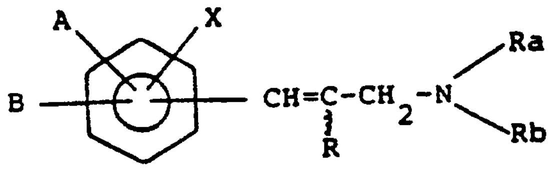

(I)

wherein:

- X is OH or

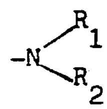

wherein $R_1$ and $R_2$ being the same or different are linear or branched $C_2$-$C_6$-alkyl, $C_3$-$C_6$ cycloalkyl, cyclopropylmethyl, benzyl, hydroxyethyl, chloroethyl or groups $R_1$ and $R_2$ taken together with the nitrogen atom, are a piperazin-1-yl, 4-N-acetyl-piperazin-1-yl, N-methyl-piperazin-1-yl or aziridinyl residue;
- R is selected in the group of hydrogen, $C_1$-$C_4$-linear or ramified alkyl, cyano or carboxyl esterified group;
- A and B being the same or different are selected in the group of hydrogen, $C_1$-$C_4$-linear or branched alkyl, $C_1$-$C_2$-alkoxy, halogen, 1-imidazolyl or a group of formula

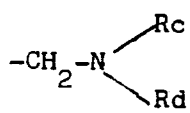

;

- Ra, Rb, Rc and Rd, which are the same or different, are selected in the group of $C_1$-$C_4$-linear or branched alkyl, $C_3$-$C_6$-cycloalkyl, cyclopropylmethyl, hydroxyethyl, chloroethyl, groups of formula

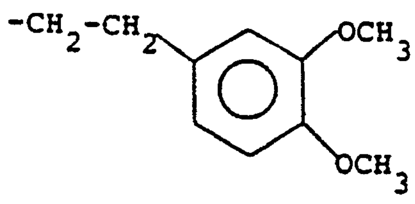

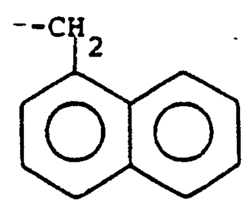

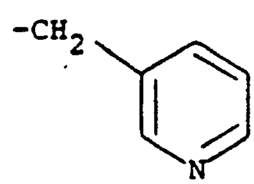

or the substituents of an amino disubstituted group, taken together with the nitrogen atom represent a saturated or unsaturated nitrogen heterocyclic ring selected from the group formed by morpholin-1-yl; pyrrolidin-1-yl, piperidin-1-yl, piperazin-1-yl, N-methyl-piperazin-1-yl, N-p-fluorophenyl-piperazin-1-yl, N-(phenylthiomethyl)piperazin-1-yl, N-(bisphenylmethyl) piperazin-1-yl, N-(bis-p-fluorophenylmethyl)-piperazin-1-yl, aziridinyl, 2-carboxy-pyrrolidin-1-yl, 2-cyano-pyrrolidin-1-yl, 3-thiazolidinyl, 4-carboxy-3-thiazolidinyl.
- with the condition that the substituent X and the propenyl chain will be in ortho and para positions, and that the substituents A and B will be in the free ortho and para positions of the ring, as specified in formulae Ia and Ib:

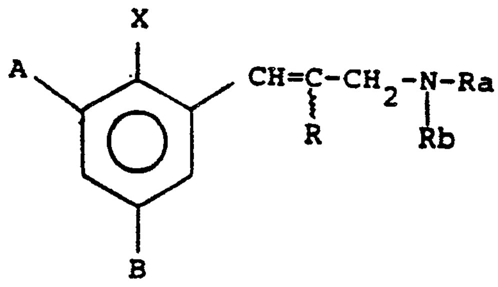

(Ia)

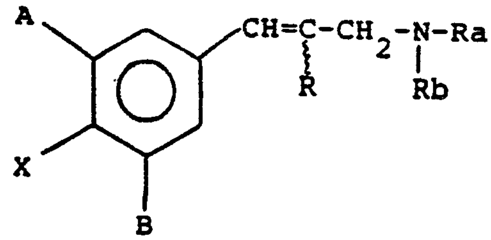

(Ib)

with the proviso that when $-NR_aR_b$ is N-(bis-p-fluorophenylmethyl)piperazin - A or B or both must be the group $-CH_2-NR_aR_b$.

Objects of the present invention are also salts of compounds of formula I with acids acceptable for pharmaceutical and veterinary use, and are also object of the present invention, like the geometric cis- and trans-isomers and mixtures thereof.

Examples of pharmaceutically acceptable salts include chlorhydrates, bromhydrates, iodhydrates, alkyl and aryl sulphates, phosphates, sulphates, maleates, fumarates, succinates, tartrates, citrates and other salts of common use in the art.

Some of the salts of the invention have sometimes particular advantages due to an increased solubility, an increased or lowered stability, possibility of crystallization, no disgusting taste, etc. , but all these respects are secondary in comparison with the main physiological action of the free base that does not depend on the kind of acid used.

In the formulae of the present invention the bond lines

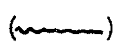

indicate that the relating substituents have no defined stoichiometrical identity, i.e. they indicate that the R group may be in cis- or trans-position with respect to the aromatic ring.

Preferred compounds of the invention are those wherein:

- X is OH;
- A and B are hydrogen, methoxy, bromo, fluoro or

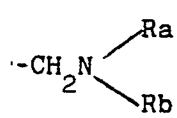

- R is hydrogen, cyano or methyl.

Particularly preferred compounds are those wherein:

- X is a group

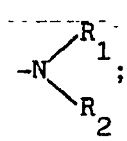

- A and B are hydrogen, R is cyano, hydrogen or methyl.

Specific examples of preferred compounds of the invention are those of formula Ib

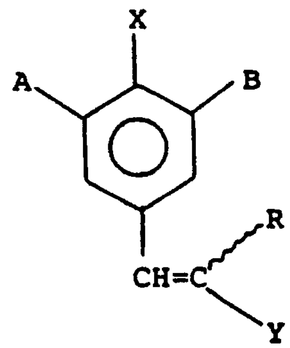

(Ib)

wherein the variants X, A, R, B and Y have the following meanings:

1) X = OH, A = $OCH_3$, R = H, B and Y = 1-morpholinomethyl;
2) X = OH, A = $OCH_3$, R = H, B = H, Y = 1-morpholinomethyl;
3) X = OH, A = $OCH_3$, R = H, B = $OCH_3$, Y = 1-morpholinomethyl;
4) X = OH, A = $OCH_3$, R = H, B and Y = 1-pyrrolidinylmethyl;
5) X = OH, A = $OCH_3$, R = H, B = $OCH_3$, Y = piperazinomethyl;
6) X = OH, A = $OCH_3$, R = H, B = $OCH_3$, Y = 3-thiazolidinylmethyl;
7) X = OH, A = $OCH_3$, R = H, B = 4-morpholinomethyl, Y = 1-prolinylmethyl;
8) X = OH, A= $OCH_3$, R = $CH_3$, B = $OCH_3$, Y = 1-morpholinomethyl;
9) X = OH, R = H, A, B and Y = 1-pyrrolidinomethyl;
10) X = OH, A = H, R = CN, B and Y = 1-morpholinomethyl;
11) X = OH, A = $OCH_3$, R = H, B = 1-pyrrolidinomethyl, 1 = 4-(bis-p-fluorophenylmethyl)-1-piperazinomethyl;
12) X = OH, A = $OCH_3$, R = H, B = 1-morpholinomethyl, Y = 4-(bis-p-fluorophenylmethyl)-1-piperazinomethyl;
13) X = OH, A = $OCH_3$, R = H, B = $OCH_3$, Y = 4-(bis-p-fluorophenylmethyl)-1-piperazinomethyl;
14) X = OH, A = $OCH_3$, R = CN, B = $OCH_3$, Y = [2-(3,4-dimethoxyphenyl)-ethyl]-methyl-amino-methyl;
15) X = OH, A = $OCH_3$, R = H, B and Y =4-(bis-p-fluorophenylmethyl)-1-piperazinomethyl;
16) X = OH, A and B = 1-pyrrolidinomethyl, R = H, Y = 4-(bis-p-fluorophenylmethyl)-1-piperazinomethyl;
17) X = OH, A and B = 1-morpholinomethyl, R = H, Y = 4-(bis-p-fluorophenylmethyl)-1-piperazinomethyl;
18) X = OH, A = $OCH_3$, R = H, B = Br, Y = 4-(bis-p-fluorophenylmethyl)-1-piperazinomethyl;
19) X = $N(Et)_2$, A = H, R = H, B = H, Y = 4-(bis-p-fluorophenylmethyl)-1-piperazinomethyl;
20) X = $N(Et)_2$, A = H, R = H, B = H, Y = 1-morpholinomethyl;
21) X = OH, A = $OCH_3$, R = H, B and Y = N-methyl-piperazinomethyl;
22) X = OH, A = $OCH_3$, R = H, B and Y = N-p-fluorophenyl-piperazinomethyl;
23) X = OH, A = $OCH_3$, R = H, B and Y = N,N-diethanolaminomethyl;
24) X = OH, A = $OCH_3$, R = H, B and Y = N,N-di-(2-chloroethyl)aminomethyl;
25) X = OH, A = B = 1-pyrrolidinomethyl, R = H, Y = 1-prolinylmethyl.

Examples of preferred compounds of formula Ia

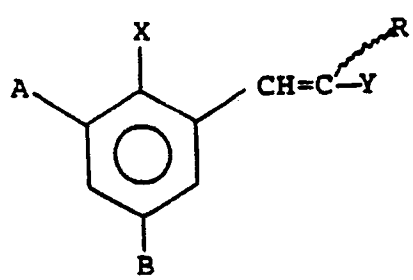

(Ia)

are those wherein the variants A, B, X, R and Y have the following meanings:

1) X = OH, A = H, B = F, Y = $-CH_2-N(Et)_2$, R = H;
2) X = OH, A = $OCH_3$, B = H, Y = 1-morpholinomethyl, R = H;
3) X = OH, A = $OCH_3$, B = Y = 1-morpholinomethyl, R = H;
4) X = OH, B = F, A = Y = 1-morpholinomethyl, R = H;
5) X = OH, A = $OCH_3$, B = H, R = H, Y = 4-(bis-p-fluorophenylmethyl)-1-piperazinomethyl;
6) X = OH, A = $OCH_3$, R = H, B = Y = 4-(bis-p-fluorophenylmethyl)-1-piperazinomethyl;
7) X = OH, A = $OCH_3$, B = 1-morpholinomethyl, R = H, Y = 4-(bis-p-fluorophenylmethyl)-1-piperazinomethyl;
8) X = OH, A = B = 1-morpholinomethyl, R = H, Y = 4-(bis-p-fluorophenylmethyl)-1-piperazinomethyl;
9) X = $N(Et)_2$, A = B = R = H, Y = 4-(bis-p-fluorophenylmethyl)-1-piperazinomethyl;
10) X = OH, A = $OCH_3$, R = $CH_3$, B = Y = 1-morpholinomethyl;
11) X = OH, A = Y = 1-morpholinomethyl, B = $OCH_3$, R = H;
12) X = OH, A = Y = 1-morpholinomethyl, B = $OCH_3$, R = H;
13) X = OH, A = $OCH_3$; B = Br, Y = 1-morpholinomethyl, R = H;
14) X = OH, A = $OCH_3$, B = 1-imidazolyl, Y = 1-morpholinomethyl, R = H;
15) X = OH, A = $OCH_3$, B = R = H, Y = N-methylpiperazinomethyl;
16) X = OH, A = $OCH_3$, B = R = H, Y = N-2-hydroxyethyl-1-piperazinomethyl;
17) X = OH, A = $OCH_3$, B = R = H, Y = N-methyl-N-cyclohexylaminomethyl;
18) X = OH, A = Y = N,N-di(2-chloroethyl)-aminomethyl, B = R = H.

In Arch. Pharm. 1972, 305, S. 610 ff. are described some cinnamylamines, substituted in p-position by $-N(CH_3)_2$, but without any pharmacological properties.

The 4-hydroxy metabolite of fluranizine is described in Drug Research 33, 1983, p. 1142 ff. Pharmacological properties and a synthesis thereof is not disclosed.

Compounds of the present invention are prepared by reacting an acid of formula II or one of its salts

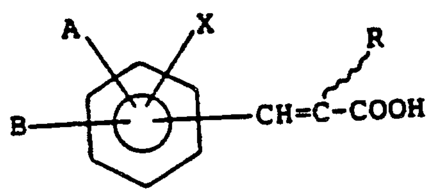

(II)

wherein A, B, X and R are as above defined, with formaldehyde and a secondary amine of formula III

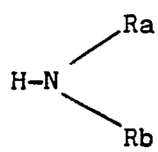

(III)

wherein Ra and Rb are as above defined. Formation of cinnamylamines substituted by reaction of cinnamic acids with formaldehyde and amines has never been described up to now, and it is thus object of the present invention.

Reaction of acids II with formaldehyde and amines III is carried out by dissolving in suitable solvent formaldehyde and amine III generally in equimolecular quantities or using a molar excess of 2:1 or 3:1 of formaldehyde with respect to amine, together with such a quantity of acid II that the rate between moles of acid II and moles of amine III is included between 1:1:1 and 1:6. The resulting solution is then kept at a temperature ranging from room temperature and the solvent's reflux temperature. Suitable solvents for effecting the reaction include water, aqueous solutions of alkaline bicarbonates (such as $LiHCO_3$, $NaHCO_3$, $KHCO_3$), alcohols that can be mixed with water (such as ethanol, methanol, propanol and isopropanol), dimethoxyethane, tetrahydrofurane, diglime and mixtures thereof.

When the reaction is carried out in water or in aqueous solutions of alkaline bicarbonates, the reaction temperature ranges from room values and $50\,^{\circ}C$, but generally it is carried out at room temperature. In these conditions reaction time ranges from few minutes to 12 hours, but generally, the reaction is completed in 30'.

When the reaction is carried out in alcohols or ethers,or mixtures thereof with water, reaction temperature may vary from room values to the solvent's reflux temperature, but generally the reaction is

carried out at 75° C. Reaction time ranges from a few minutes to 24 hours, but normally reaction is completed in two hours.

Experimental conditions used in reaction of acids II with formaldehyde and secondary amines III to give compounds of formula I are the same as for reaction of aminomethylation of phenols,that on its turn is a typical example of Mannich reaction. When the reaction is carried out using as starting material acids II wherein X is OH and at least one of A and B is hydrogen, it is possible, by varying quantities of reagents and experimental modalities, to make the reaction produces compounds I of different structure. When, for example the reaction is carried out on an acid of formula II wherein A and B are different from hydrogen, the molar ratio between amine III and acid II necessary to complete reaction varies from 1:1 to 1:5:1. Generally the reaction is completed utilizing a 10% molar excess of amine with respect to the acid.

When the reaction is carried out using as starting material an acid II in which at least one of A and B is hydrogen and X is OH, it is possible, if desired, to avoid aminomethylation of aromatic ring, using equimolecular quantities of acid II, amine III and formaldehyde, and adding slowly the formaldehyde to the solution of acid II and amine III. If it is used as starting material an acid of formula II wherein at least one of A and B is hydrogen, with the intention to obtain products of formula I from the reaction, wherein at least one of A and B is

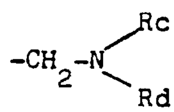

and Rc and Rd are the same as Ra and Rb, the reaction itself is carried out using an excess of formaldehyde and amine III with respect to acid II in a ratio varying from 3:1 to 6:1. When the reaction is carried out using acids of formula II as starting material, wherein X is

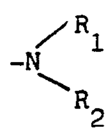,

and $R_1$ and $R_2$ are as above defined, it is preferably used a molar excess of 10% of amine III.

Acids of formula II wherein A and B are different from

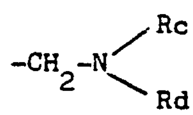

are known products and they are prepared following known methods. In particular, acids of formula II wherein A and B are different from

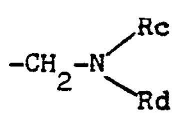

and X is OH, are prepared in accordance with J.F.E. Dupin and J. Chenault in Synthetic Communication, Vol. 15 (7), pages 581-586, 1985. Compounds of formula II wherein X is

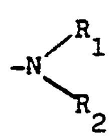

are prepared according to Gensler and Berman, in J. Am. Chem. Soc., Vol. 80, pages 4949-4954, 1958.

Compounds of formula II wherein X is OH and at least one of A and B is

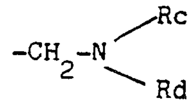

are obtained by alkaline hydrolysis from esters of formula IV

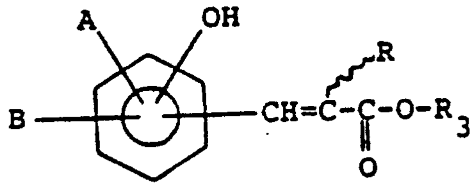

(IV)

wherein R is as above defined, but it is different from esterified carboxyl, $R_3$ is methyl or ethyl and one of A and B is a group of formula

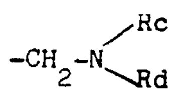

wherein Rc and Rd are as above defined.

If desired, acids of formula II wherein at least one of A and B is

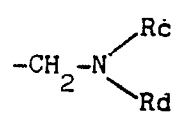

may be reacted without being isolated under form of alkaline or alkaline-earth salts with formaldehyde and a secondary amine of formula III to give compounds of formula I wherein at least one of A and B is

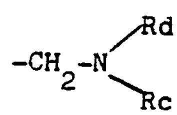

while the other is as above defined and wherein the amino group

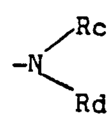

may be different from aminogroup

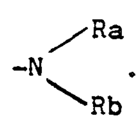

Hydrolysis reaction of esters of formula IV is effected in aqueous alcohol solutions such as methanol, ethanol, isopropanol in presence of a molar excess of an alkaline or alkaline-earth hydroxide, for example NaOH, KOH, $Mg(OH)_2$, $Ca(OH)_2$ or mixtures thereof.

The molar excess of alkaline or alkaline-earth hydroxyde with respect to ester varies from 2:1 to 4:1.

The reaction is carried out at temperatures varying from room temperature to the solvent's reflux temperature. Reaction times range from few hours to 48 hours. Generally the reaction is executed in an aqueous ethanol solution at 50° C using a molar excess of NaOH of 2:1 with respect to ether of formula IV and the reaction is usually completed in 4 hours.

Esters of general formula IV, wherein R is CN are prepared by reacting the aldehydes of general formula V

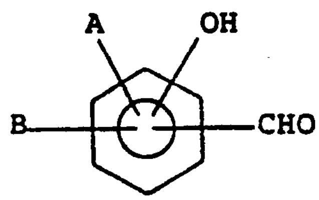

(V)

wherein A and B are as above defined, with ethyl cyano-acetate according to Beilstein 10 : 520.

Finally, esters of general formual IV wherein R is $C_1$-$C_4$-alkyl are prepared by reaction of an aldehyde of formula V with a stabilized ylide of formula VI

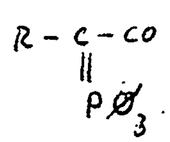

(VI)

wherein R and $R_3$ are as above defined.

Reaction of an aldehyde of formula V with stabilized ylides VI is a typical Wittig reaction. It is carried out in presence of molar quantities quite similar to reagents V and VI in a temperature ranging from -30° C to the solvent's reflux temperature but preferably between -10° C and room temperature, and in solvents such as tetrahydrofurane, dimethoxyethane, dimethylformamide, dimethylsulphoxide, benzene, toluene or mixtures thereof. The stabilized ylides are otabined by means of action of bases, such as sodium-hydroxide, etc. on the corresponding phosphonium salt.

The aldehydes of formula V are products known in literature and may be obtained following known methods.

Particularly, the preparation of aldehydes of formula V is described in the patent appln. No.21433 A/86 of 7th Aug.1986.

When compounds of the invention are administered by oral, intraperitoneal and intravenous route to rats and mice, they show low toxicity, with $LD_{50}$ after oral administration included between 0.3 g and 0.9 g/Kg.

It is described a reduced production of malondialdehyde after stimulation with $H_2O_2$ of erythrocyte membranes of rats incubated with compounds of the invention.

When administered by oral and intraperitoneal route, compounds of the invention further protect mice from sudden death induced by administration (in bolus) or arachidonic acid or mixtures of ADP and collagene.

Compounds of the invention are moreover able to protect cell membranes from oxidation,thus preventing lipoperoxidation phenomena.

Compounds of the invention are moreover able to interfer with intra- and extracellular oxidation processes, thus a control of the same is enabled and they act as "radical scavenger" of free radicals.

Trimetazidine, for instance, is a molecule provided with anti-angina activity, but without calcium-blocking activity, and its mechanism of action has not yet been well defined.

Recent studies show that its pharmacological activity is due to a metabolite of the substance (probably having a phenolic nature) that is able to protect cell membranes from damages caused by oxygen free radicals. Cynnarizine and flunarizine are substances with cinnamylamine structure, vinyl analogues or trimetazidine, that are active as vasodilators and calcium blocking agents, and used as brain vasodilators, with protecting effect on brain. Since it seems that generation of free radicals, together with the subsequent peroxidative degeneration of cell-membrane is responsible for the brain-ischemic damage, the anti-oxidation or as "radical-scavenger" activity of flunarizine has been valued in vitro (Arch. Int. Pharmacology, 272, 283, 1984). Flunarizine resulted to be very active as "radical scavenger", thus differing from other calcium antagonists, like nifedipine, that, on their turn, are completely lacking of brain-protecting activity.

Successively it has been found that compounds of the invention have a high anti-oxidation or free-radical scavenger activity, comparable and sometimes higher than that of known anti-oxidation agents, like α-tocopherol and ascorbic acid.

Potential as "radical scavengers" of compounds of the invention is chemically demonstrable (see for ex. A Mellors et al, J. Biol. Chem., 241, 4353, 1966) by measuring the absorption decrease to 510 nm. of solutions 0.1 mM of a steady radical, such as diphenyl-p-picryl-hydrazyle.

According to R. Rubo et al, Arch. Int. Pharmacol., 272, 283, 1984 values of activity are expressed as IC $_{0,2}$ (cuvette concentration of examined substance M that is able to reduce of 0.2 units of optical density, absorption of a solution 0.1 mM of diphenylpicrylhydrazile).

The table reports values of $IC_{0.2}$ for some of the compounds of the invention, for some known anti-oxidation agents, ascorbic acid, hydroquinone and for a known cinnamylamine, flunarizine.

| Compound | $IC_{0,2}$ |
|---|---|
| 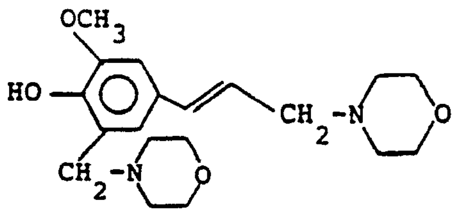 | 8 |
| 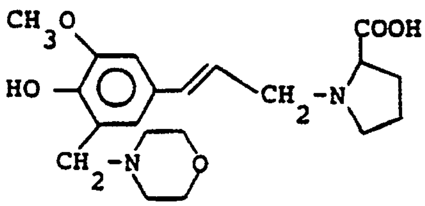 | 6 |
| 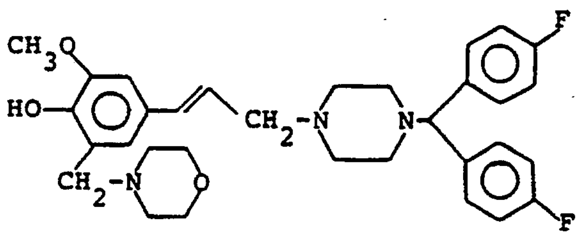 | 13 |
| 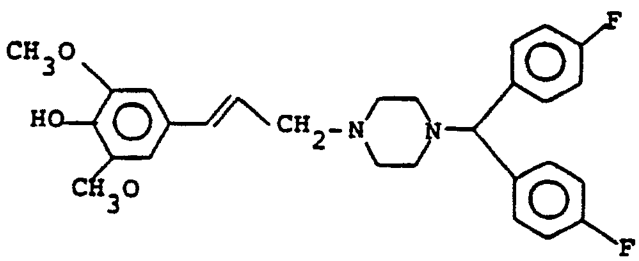 | 7 |
| Hydroquinone | 9,5 |
| Ascorbic acid | 11,5 |
| Flunarizine HCl | 160 |

Data reported in the Table indicate clearly that some of the compound have an anti-oxidation activity in vitro comparable or higher than that of ascorbic acid and hydroquinone.

Compounds of the invention have also a radical scavenger and anti-oxidation activity 10 times superior to that of flunarizine whose anti-oxidation activity seems to be bound to brain protecting action of flunarizine.

Compounds of the present invention are able to modify intra- and extracellular availability of $CA^{++}$ ions

in mammals biological liquids.

For this reason compounds of the invention are therapeutically useful to control tissue metabolic processes, aiming at the regulation of contractions of smooth and striated muscles.

Utility of compounds of the invention includes also control "in vivo" of enzymatic processes depending on cellular availability of $Ca^{++}$ ions, as for example inhibition and/or activation of phospholipase, phosphodiesterase, collagenase, elastase, etc. Compounds of the invention are particularly useful to control intra-and extracellular movements of electrclites ($Ca^{++}$, $Na^{+}$, $K^{+}$,$Mg^{++}$) that regulate deformation of cellular components of blood, such as platelets, leucocytes, erythrocytes, thus cooperating to blood viscosity regulation.

When tested "in vivo" compounds of the invention have a secretolitic effect on bronchial mucus, as shown by red-phenol and fluoresceine sodium salts tests on rats and mice. Compounds of the invention moreover modify physical (viscosity and volume) and biochemical parameters of mucus produced by bronchitic rabbits.

From the above arguments it is evident that compounds of the invention are useful in therapy as tissue protectors, anti-thrombotic, anti-oxidation, mucolitic agents, etc.

To reach the desired effects in human and veterinary therapy, compounds of the invention may be administered parenterally, for ex. as intravenous, hypodermic and intramuscular injection, as infusion, or by oral route. Compounds may be administered to patients under pure form or as pharmaceutical compositions.

Opportune pharmaceutical compositions may be realized in accordance with known techniques, as described for ex. in "Remington's Pharmaceutical Sciences Handbook",Hack Publishing Co.U.S.A.

When compounds of the invention are used as antihypertensive agents, dosage will vary according to seriousness of hypertension and route of administration.

Quantity of active principle administered by oral route may range from 0.01 mg/kg/day to 100 mg/kg/day, preferably from 0.5 mg/day to 10 mg/kg/day.

Quantity of active principle administered by perenteral route may contain, for ex., from 0.001 mg/kg/day to 10 mg/kg/day, preferably from 0.01 mg/kg/day to 1 mg/kg/day.

A dose for oral administration may contain, for example, from 0.1 to 1000 mg of active principal.

Compounds of the invention may be administered once a day, but more spaced and/or repeated administrations may be convenient in some cases and may vary according to conditions of the patient, routes of administration and dosages used. In the present occasion the word "patient" means hot-blooded animal,man included.

For oral administration compound may be formulated in solid or liquid preparations as capsules,pills, tablets, suspensions or emulsions. Solid unit dose may be a hard or soft gelatine capsule containing lubricants or inert excipients such as lactose, saccharose or amide.

Compounds of the invention may also be formulated as tablets utilizing conventional excipients like lactose, saccharose, amide, gelatine, alginic acid,stearic acid, magnesium stearate,etc.

For parenteral administration compounds may be prepared in injectable formulations by melting or suspending them in physiologically acceptable diluents, with a vehicle that can be sterile water or oil, with or without adding other excipients. Oils employed may be of vegetable, animal, mineral or synthetic origin, like peanut, soya and mineral oil. Generally, as a vehicle for injectable solutions it is possible to utilize water, aqueous solutions of mineral salts, aqueous solutions of dextrose or other sugars,ethanol glycols such as propylene or polyethylene glycols.

Compounds may also be administered by oral route, as suppositories, mixed with conventional vehicles as for example cocoa butter, wax, polyvinylpyrrolidone or polyoxyethylenglycole or derivatives thereof.

The preferred administration route for compounds of the invention is the oral route.

The invention is further described but not limited by the following examples.

**EXAMPLE 1**

A solution of methyl 4-hydroxy-5-methoxy-3-(1-pyrrolidinylmethyl)-cinnamate (2.77 g) and sodium hydrate (0.76 g) in 95% ethanol (60 ml) and water (10 ml) is heated to reflux for 90′. After cooling at 0 °C, pH of the solution is brought to 7.2 with concentrated hydrochloric acid (2.8 ml) and formaline at 37% (0.92 ml) is added. The solution is warmed at reflux temperature and successively added in 40:50′ by a solution of 1-[bis-(p-fluorophenyl)methyl]piperazine (3.28 g) in ethanol (50 ml).

The reflux is continued for one hour. Successively the solvent is evaporated under reduced pressure and the obtained residuate is dissolved in ethyl acetate (80 ml), the resulting suspension is filtered and the filtrate is anhydrified on $Na_2SO_4$. The organic phase is concentrated and the residue is chromatographated

on $SiO_2$; eluent ethyl acetate/n-hexane/triethylamine 9/3/0.3. G. 2.6 of 4-[bis-(p-fluorophenyl-methyl]-[[4-hydroxy-5-methoxy-3-(1-pyrrolidinylmethyl)]-cinnamyl]-piperazine are obtained that are dissolved in ethyl ether (25 ml) and treated with a solution 8.4 N of HCl in isopropanol (3.2 ml) to give 2.74 g of the corresponding hexahydrate trichlorhydrate; m.p.174-188° C.

## EXAMPLE 2

Using in procedure of Example 1 the opportunely substituted cinnamic esters, or the opportune amines, the following compounds are prepared:

- 4-[bis(p-fluorophenyl)-methyl]-1-[[4-hydroxy-5-methoxy-3-(4-morpholinylmethyl)]-cinnamyl]-piperazine, 2HCl, 2.5 $H_2O$, m.p. (decomposition);
- 4-[bis(p-fluorophenyl)-methyl]-[[2-hydroxy-3-methoxy-5-(4-morpholinylmethyl)]-cinnamyl]-piperazine trichlorhydrate; m.p.177-190° C alteration; 200-204° C decomposition;
- 4-[bis(p-fluorophenyl)-methyl]-1-[[4-hydroxy-3,5-bis(4-morpholinylmethyl)]-cinnamyl]-piperazine; m.p.148-150° C; bitartrate, bihydrate; m.p. 90-93° C;
- 4-[bis(p-fluorophenyl)-methyl]-1-[[4-hydroxy-3,5-bis(4-pirrolidinylmethyl)]-cinnamyl]-piperazine; m.p.131-133° C, tetrachlorhydrate monohydrate; m.p.196-200° C;
- 4-[bis(p-fluorophenyl)-methyl]-1-[[2-hydroxy-3,5-bis(4-morpholinylmethyl)]-cinnamyl]-piperazine; NMR ($CDCl_2$, TMS): $\delta$ = 3.20, d, 2H (Ar-CH=CH-$\underline{CH_2}$-N); $\delta$ = 4.25, s, 1H (N-$\underline{CH}$-$Ar_2$); $\delta$ = 6.11-7.65,m, 12H (aromatic hydrogens + $\overline{\text{-CH=CH-}}$);
- 1-[[4-hydroxy-5-methoxy-3-(4-morpholinylmethyl)]-cinnamyl]-proline; sesquihydrate; m.p.70-72° C (decomposition);
- N-[[4-hydroxy-5-methoxy-3-(4-morpholinylmethyl)]-cinnamyl]-N-methyl-cyclohexylamine; NMR ($CDCl_2$; TMS): $\delta$ 0.82-2.1, m,11H, (-N-cyclohexyl);$\delta$=2.2, s, 3H (-N-$CH_3$); $\delta$ = 3.15,d,2H (CH=CH-$\underline{CH_2}$):$\delta$ = 5.8-7.2, m, 4H (aromatic + -CH=CH-);
- N-[[4-hydroxy-5-methoxy-3-(4-morpholinylmethyl)]-cinnamyl]-N-methyl-homoverartrilamine; NMR ($CDCl_3$; TMS):$\delta$ = 2.35,s,3H (N-$CH_3$); $\delta$ = 3.18,d,2H (-CH=CH-$\underline{CH_2}$-N-):$\delta$ = 5.70-6.90, m,7H (aromatic+-CH=CH).

## EXAMPLE 3

A solution of 1-[bis-(p-fluorophenyl)-methyl]-piperazine (g 0.28) is added in a period of 15' to a boiling solution of 4-hydroxy-5-methoxy-3-[4-[bis-(p-fluorophenyl)-methyl]-piperazin-1-yl-methyl]-cinnamic acid (0.44 g) and formaline 37% (0.08 ml) in ethanol (5 ml). When adding is completed, the heating is prosecuted for 90'. Successively,the solvent is evaporated under a reduced pressure and the obtained residue is dissolved in ethyl acetate (15 ml). After washing with $NaHCO_3$ 5% (5 ml) and with a saturated solution of NaCl the organic phase is anhydrified ($Na_2SO_4$) and the solvent is evaporated under reduced pressure. The obtained residuate is purified by chromatography on column ($SiO_2$; eluent ethyl acetate /n-hexane/TEA 10/10/1). G. 0.45 of 4-[bis-(p-fluorophenyl)-methyl]-1-[[4-hydroxy-5-methoxy-3-[4-bis-(p-fluorophenyl)-methyl]-piperazin-1-yl-methyl]-cinnamyl]-piperazine are obtained, and when they melted in ethyl acetate (10 ml) and heated with a solution 8.4 N of HCl in isopropanol (0.6 ml) they give 0.52 g of the corresponding bihydrate tetrachlorhydrate; m.p.162-170° C (decomposition).

## EXAMPLE 4

A solution of 4-hydroxy-3-methoxy-cinnamic acid (1.55 g) and N-methylpiperazine (2.21 ml) in water (30 ml) is added with formaline 37% (1.62 ml). It is left at room temperature for 12 hours. Successively 5% $NaHCO_3$ (10 ml) is added and the solution is extracted with chloroform. The organic extracts are put together and washed with NaCl saturated solution (10 ml), anhydrified ($Na_2SO_4$) and the solvent is evaporated under a reduced pressure.

The obtained residue is crystallized by ethyl ether/ethyl acetate. G. 2.49 of 4-methyl-1-[[4-hydroxy-5-methoxy-3-[methyl-piperazin-1-yl-methyl]-cinnamyl]-piperazine,m.p.118-121° C.

## EXAMPLE 5

A solution of 4-hydroxy-3-methoxy-cinnamic acid (7.76 g) morpholine (8.78 g) and formaline 37% (8.11 ml) in ethanol (50 ml) is heated to reflux for an hour. The solvent is thus separated under reduced pressure and the obtained residue is partioned between $NaHOC_3$ 5% (20 ml) and ethyl acetate (100 ml). The organic

phase is separated and the aqueous one is re-extracted with ethyl acetate (2 x 70 ml). Organic extracts together are washed with NaCl saturated solution (20 ml), anhydrified ($Na_2SO_4$) and the solvent is evaporated under a reduced pressure. The obtained residue is crystallized from ethyl ether (200 ml). G. 11.08 of 4-[[4-hydroxy-5-methoxy-3-(4-morpholinylmethyl)]-cinnamyl]-morpholine,m.p.101-103 ° C, are obtained.

## EXAMPLE 6

Using in the procedures of examples 3, 4, 5 the cinnamic acids opportunely substituted and the opportune amines, the following compounds were obtained:

- 4-[[2-hydroxy-3-methoxy-5-(1-morpholinylmethyl)]-cinnamyl-morpholine, m.p.111-112 ° C;
- 4-[[2-hydroxy-5-methoxy-3-(1-morpholinylmethyl)]-cinnamyl]-morpholine bichlorhydrate bihydrate,m.p.135-140 ° C (decomposition;
- 4-[[4-hydroxy-3,5-dimethoxy]-cinnamyl]-morpholine monochlorhydrate, m.p.178-182 ° C;
- 4-[[2-hydroxy-5-fluoro-3-(1-morpholinylmethyl)]-cinnamyl-morpholine, m.p.162-164 ° C;
- 1-[[4-hydroxy-5-methoxy-3-(1-pyrrolidinylmethyl)]-cinnamyl]-pyrrolidine bichlorhydratesesquihydrate, m.p.95 ° C (decomposition);
- 1-[[4-hydroxy-3,5-bis-(1-pirrolidinylmethyl)]-cinnamyl]-pirrolidine hdyrate, m.p.79 ° C (decomposition);
- 4-[bis-(p-fluorophenyl)-methyl]-1-[[4-hydroxy-3,5-dimethoxy]-cinnamyl]-piperazine bichlorhydrate hemihydrate,m.p.213-216 ° C;
- 4-[bis-(p-fluorophenyl)-methyl]-1-[[2-hydroxy-3-methoxy-5-[bis-(p-fluorophenyl)-methyl]-piperazine-1-yl-methyl]]-cinnamyl]-piperazine tetrachlorhydrate bihydrate, m.p.184.7-186.6 ° C;
- 4-(p-fluorophenyl)-1-[[4-hydroxy-5-methoxy-3-[4-(p-fluorophenyl)-piperazine-1-yl-methyl]]-cinnamyl]-piperazine m.p.149-152 ° C;
- 3-[(4-hydroxy-3,5-dimethoxy)-cinnamyl]-piperazine, m.p.120-121 ° C;
- N-[[2-hydroxy-5-fluoro-3-(diethylaminomethyl)]-cinnamyl]-N,N-diethylamine; NMR ($CDCl_2$; δ TMS); δ 1,10, t,12H ( $CH_3$-$CH_2$-N-$CH_2$-$CH_3$) 2.62,q,8H ($CH_2$-$CH_2$-N-$CH_2$-$CH_3$); δ3,31, d,2H ($A_2$-CH = CH-$CH_2$-N); 3.70, s,2H, (Ar-$CH_2$-N-);δ 5.9-7.28,m,4H (aromatics + -CH=CH-);δ 10.22, s, 1H (-O-H);
- N-[[4-hydroxy-5-methoxy-3-[bis-(2-hydroxyethylamino)-methyl]]-cinnamyl]-diethanolamine; NMR ($CDCl_2$, TMS); 2.68, y,

8H ( 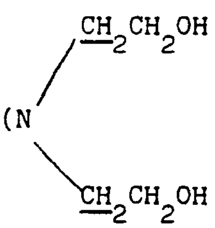 ); δ 3.28,d,2H (-CH=CH-$CH_2$-N); δ 3.6, t,

8H ( 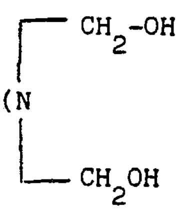 ); δ 4.25, s, 2H (A-$CH_2$-N); δ 5.70-6.99, m,

4H (aromatics = -CH=CH-);

- 4-(3,5-dibromo-2-hydroxy)-cinnamyl]-morpholine;
- 4-[bis-(p-fluorophenyl)-methyl]-1-[[3-bromo-4-hydroxy-5-methoxy]-cinnamyl]-piperazine;
- 4-[(5-bromo-2-hydroxy-3-methoxy)-cinnamyl]-morpholine;
- 4-[[2-hydroxy-5-(1-imidazolyl)-3-methoxy]-cinnamyl]-morpholine.

## EXAMPLE 7

A suspension of 3,5-dimethoxy-4-hydroxy-cinnamic acid (4.48 g) in water (50 ml) is added with N-formyl-piperazine (2.5 ml). The obtained solution is added at 20 ° C with a formaline 37% solution (3.9 ml) in water (15 ml) in a period of 30'. After 12 hours at room temperature, the reaction mixture is poured in an

excess of $NaHCO_3$ 5%, saturated with NaCl and extracted with chloroform (5 x 30 ml). The organic extracts together are anhydrified ($Na_2SO_4$) and the solvent is evaporated under a reduced pressure. The obtained residue is melted in methanol (90 ml), concentrated hydrochloric acid (8 ml) is added and the solution is heated to reflux for 4 hours. After a night at room temperature the compound is diluted with ethyl ether (90 ml). By cooling at 0 °C a separation of solid is obtained, which is separated by filtration under nitrogen atmosphere and washed with ethyl ether. G. 3.5 of 1-[4-hydroxy-3,5-dimethoxy)-cinnamyl]-piperazine bichlorhydrate monohydrate, m.p. 168-171 °C are obtained.

**EXAMPLE 8**

Using in procedure of example 7 opportunely substituted cinnamic acids and N-formyl-piperazine, the following compounds are obtained:

- 1-[(5-bromo-2-hydroxy-3-methoxy)-cinnamyl]-piperazine;
- 1-[(2-hydroxy-5-(1-imidazolyl)-3-methoxy-cinnamyl]-piperazine;
- 1-[(3-bromo-4-hydroxy-5-methoxy)-cinnamyl]-piperazine.

**EXAMPLE 9**

A boiling solution of 2-hydroxy-3-methoxy-cinnamic aicd (13.6 g) and morpholine (8.52 ml) in n-propanol (130 ml) is added slowly with a formaline 37% solution (7.94 ml) in water (20 ml). When adding is finished the compound is left to reflux for 2 hours and then at room temperature for one night. The solvent is evaporated under reduced pressure, and the obtained residue is dissolved in ethyl acetate (130 ml) and washed with $NaHCO_3$ 5% (2 x 20 ml) and with NaCl saturated solution (30 ml). The organic phase is anhydrified ($Na_2SO_4$) and the obtained residue after evaporation of the solvent under reduced pressure, is purified by chromatography on column ($SiO_2$, ethyl acetate / TEA 20/1).

G . 11.28 of 4-[(2-hydroxy-3-methoxy)-cinnamyl]-morpholine m.p.139-141 °C are obtained, they are melted in ethyl acetate and heated with a 8.4 N solution of HCl in isopropanol to give g 11.06 of the corresponding monochlorhydrate, m.p.187.7)188.6 °C.

**EXAMPLE 10**

Using in procedure of example 9 the cinnamic acids opportunely substituted and the opportune amine, the following compounds were prepared:

- 4-[(4-hydroxy-3-methoxy)-cinnamyl]-morpholine monochlorhydrate, m.p.182-185 °C;
- N-[(2-hydroxy-5-fluoro)-cinnamyl]-N,N-diethylamine monochlorhydrate, m.p.140-147 °C;
- 4-[bis-(p-fluorophenyl)-methyl]-1-[(2-hydroxy-3-methoxy)-cinnamyl]-piperazine, m.p.138-140 °C bichlorhydrate monohydrate, m.p.217 °C (decomposition);
- 4-methyl-1-[(2-hydroxy-3-methoxy)-cinnamyl]-piperazine;
- 4-(2-hydroxyethyl)-1-[(2-hydroxy-3-methoxy)cinnamyl]-piperazine;
- N-methyl-N-[(2-hydroxy-3-methoxy)-cinnamyl]-cyclohexylamine.

**EXAMPLE 11**

A solution of 3,5-dimethoxy-4-hydroxy-α-methyl cinnamic acid (1.14 g) and morpholine (0.46 ml) is added with 37% formaline (0.43 ml). The solution is left for 18 hours at room temperature and then it is heated at 55 °C for 2 hours. After cooling at room temperature an excess of $NaHCO_3$ is added and the compound is extracted with ethyl acetate (3 x 10 ml). organic extracts are set together, anhydrified ($Na_2SO_4$) and the solvent is separated under reduced pressure.

G. 0.80 of 4-[[4-hydroxy-3,5-dimethoxy-α-methyl]-cinnamyl]-morpholine,m.p. 103-106 °C are obtained.

**EXAMPLE 12**

Using in procedure of Example 11 opportunely substituted methyl cinnamic acids and morpholine, the following compounds are obtained:

- 4-[[4-hydroxy-5-methoxy-3-(4-morpholinylmethyl)-α-methyl]-cinnamyl]-morpholine;
- 4-[[2-hydroxy-3-methoxy-5-(4-morpholinylmethyl)-α-methyl]-cinnamyl]-morpholine.

**EXAMPLE 13**

A solution of α-cyano-4 hydroxy-cinnamic acid (1.89 g) and morpholine (2.87 g) in ethanol (4 0 ml) is added with formaline 37% (2.68 ml). The solution is heated to reflux for 7½ hours. After one night at room temperature, the solvent is separated under reduced pressure, the obtained residue is dissolved in ethyl acetate (50 ml) and washed with $NaCOH_3$ (4 x 20 ml) and with NaCl saturated solution. The organic phase is anhydrified ($Na_2SO_4$)and evaporated.

The residue is crystallized from ethyl ether and successively recrystallized from ethanol (two times). G.0.48 of 4-[[4-hydroxy-3-(4-morpholinyl-methyl)-α-cyano]-cinnamyl]-morpholine, m.p.164.1-166°C are obtained.

## EXAMPLE 14

Using in procedure of example 13 an opportunely substituted α-cyano cinnamic acid and the opportune amines, the following compounds are prepared:

- N-[[2-hydroxy-3)methoxy-α-cyano]-cinnamyl]-N-methyl-homoveratrilamine;
- N-[[2-hydroxy-3-methoxy-5-bromo-α-cyano]-cinnamyl]-N-methyl-homoveratrilamine;
- N-[[4-hydroxy-3,5-dimethoxy-α-cyano]-cinnamyl]-N-methyl-homoveratrilamine;
- N-[[4-hydroxy-5-methoxy-3-(4-morpholinylmethyl)-α-cyano]-cinnamyl]-N-methyl-homoveratrilamine;
- N-[[2-hydroxy-3-methoxy-5-(4-morpholinylmethyl)-α-cyano]-cinnamyl]-N-methyl-homoveratrilamine.

## EXAMPLE 15

A boiling solution of p-diethylamino cinnamic acid (4.0 g) and formaline (37%,2.94 ml) in ethanol (50 ml) is slowly added with a solution of 1-[bis-(p-fluorophenyl)-methyl]-piperazine (5.68 g) in ethanol (50 ml). When adding is terminated, heating to reflux is prosecuted for two hours. Successively the solvent is evaporated under reduced pressure and the obtained residue is dissolved in ethyl acetate (80 ml), washed with $NaHCO_3$ 5% (2 x 20 ml). The organic phase is then anhydrified ($Na_2SO_4$) and the solvent is evaporated under reduced pressure. The obtained residue is crystallized from isopropyl ether. G. 4.6 of 4-[bis-(p-fluorophenyl)-methyl]-1-(p-diethylaminocinnamyl)-piperazine,m.p.92-94°C are obtained.

## EXAMPLE 16

Using in procedure of Example 15 dialkylamino - cinnamic acids opportunely substituted, and the opportune amines, the following compounds are prepared:

- 4-(p-diethylamino-cinnamyl)-morpholine;
- 4-[bis-(p-fluorophenyl)-methyl]-1-[o-diethylaminocinnamyl]-piperazine;
- 4-hydroxy-1-[3,5-dibromo-2-diethylaminocinnamyl]-piperidine;
- 4-hydroxy-N-[3,5-dibromo-2-diethylaminocinnamyl]-N-methyl-cyclohexylamine.

## EXAMPLE 17

A solution of sodium hydrate (0.49 g) in water (16 ml) is added with portions of ethyl-4-hydroxy-3-(4-morpholinomethyl)-5-methoxy-cinnamate (1.35 g) and the resulting solution is heated to 45°C for 45'. Successively the solution is cooled at room temperature and pH is brought to 4.8 with HCl 2N, then L-proline methyl ester chlorohydrate (0.75 g) and successively formaline 37% (0.41 ml) are added.

After 2 ½ hours at room temperature, $NaHCO_3$ is added to obtain a pH 7.5-8.0 and the solution is quickly extracted with chloroform (3 x 30 ml). The organic extracts together are washed with a NaCl saturated solution, anhydrified ($Na_2SO_4$) and evaporated under reduced pressure.

G. 1.0 of 1-[[4-hydroxy-5-methoxy-3-(4-morpholinylmethyl)]cinnamyl]-L-proline methyl ester are obtained.

NMR ($CDCl_2$; TMS): $\delta$ = 1.59,2.28,m,4H (proline);$\delta$ = 3.01-3.02,d,2H (CH=CH-CH₂-N); $\delta$ =3,96,s,3H (-$COOCH_3$); $\delta$ = 6,20-6.81,m,4H (aromatics = -CH=CH-).

## Claims

1. Compounds of formula I

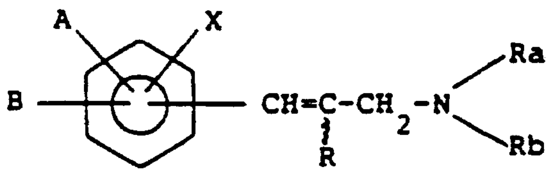 (I)

wherein:

- X is OH or

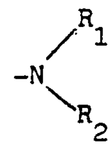

wherein $R_1$ and $R_2$ being the same or different are linear or branched $C_2$-$C_6$-alkyl, $C_3$-$C_6$-cycloalkyl, cyclopropylmethyl, benzyl, hydroxyethyl, chloroethyl or group s $R_1$ and $R_2$ taken together with the nitrogen atom, are a piperazin-1-yl, 4-N-acetyl-piperazin-1-yl, N-methyl-piperazin-1-yl, N-methyl-piperazin-1-yl or aziridinyl residuate;
- R is selected in the group of hydrogen, $C_1$-$C_4$-linear or branched alkyl, cyano or carboxyl esterified group;
- A and B being the same or different are selected in the group of hydrogen, $C_1$-$C_4$-linear or branched alkyl, $C_1$-$C_2$-alkoxy, halogen, 1-imidazolyl or a group of formula

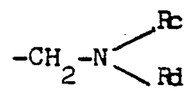 ;

- Ra, Rb,Rc and Rd, that can be the same or different are selected in the group of $C_1$-$C_4$-linear or branched alkyl, $C_3$-$C_6$-cycloalkyl, cyclopropylmethyl, hydroxyethyl, chloroethyl, groups of formula

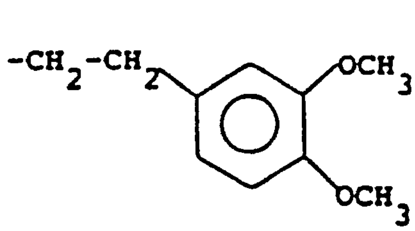

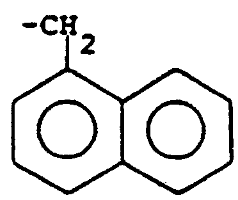

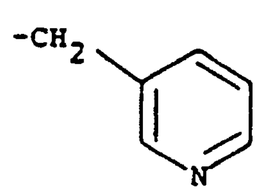

or the substituents of a disubstituted aminogroup taken together with the nitrogen atom represent a saturated or unsaturated nitrogen heterocyclic ring selected from the group formed by morpholin-4-yl, pyrrolidin- 1-yl, piperdin-1-yl, piperazin-1-yl, N-methyl-piperazin-1-yl, N-p-fluorophenyl-piperazin-1-yl, N-(phenylthiomethyl)-piperazin-1-yl, N-(bis-phenylmethyl)piperazin-1-yl, N-(bis-p-fluorophenylmethyl)-piperazin-1-yl, aziridinyl, 2-carboxy-pyrrolidin-1-yl, 2-cyano-pyrrolidin-1-yl, 3-thiazolidinyl, 4-carboxy-3-thiazolidinyl.
- with the condition that the substituent X and the propenyl chain are in orto or para position, and that the substituents A and B are in the other free orto and para positions of the ring, as specified in formulae Ia and Ib:

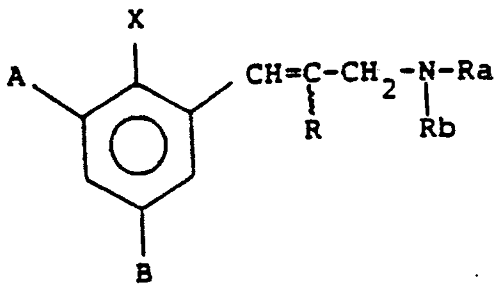

(Ia)

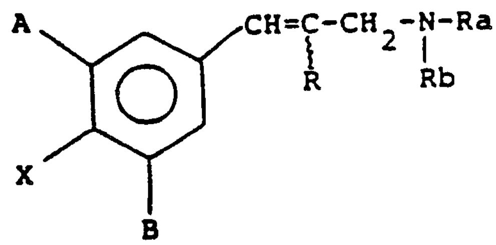

(Ib)

with the proviso that when $-NR_aR_b$ is N-(bis-p-fluorophenylmethyl)-piperazine-A or B or both must be the group $-CH_2-NR_aR_b$, their non toxic salts, optical and geometrical isomers and mixtures thereof.

2. Compounds according to claim 1, wherein X is a hydroxy group, A and B or hydrogen, methoxy, bromo, fluoro, or a residue of formula

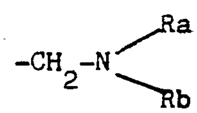

wherein Ra and Rb are the same as in claim 1.

3. Compounds according to claim 1, wherein X is a group of formula

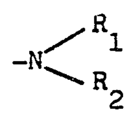

wherein $R_1$ and $R_2$ are as defined in claim 1, A and B are hydrogen and R is cyano, hydrogen or methyl.

4. Process for preparation of compounds of claims 1-3 characterized in that an acid of formula II or its sodium or potassium salt

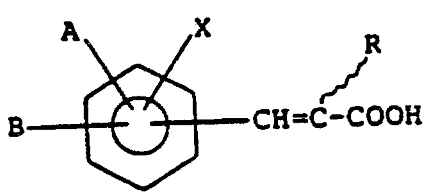

(II)

wherein A, B, X and R are as above defined, are reacted with formaldehyde and a secondary amine of formula III

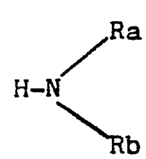

(III)

wherein Ra and Rb are as above defined.

5. Process according to claim 4, characterized in that the reaction is carried out in solvents selected in the group formed by water, aqueous solutions of alkaline carbonates or bicarbonates, lower $C_1$-$C_3$-alcohols, dimethoxyethane, tetrahydrofurane, diglime and mixtures thereof.

6. Process according to claims 4 or 5, characterized in that molar rates between acid II and amine III are substantially equivalent.

7. Process according to claims 4 or 5 characterized in that an excess of formaldehyde and amine is used with respect to acid II that may range from 3:1 to 6:1.

8. Pharmaceutical compositions containing as an active principle at least one of compounds of claims 1-3 and a pharmaceutically acceptable vehicle.

9. Compounds according to claims 1-3 used as therapeutical agents.

10. Compounds according to claims 1-3 used as radical "scavengers",anti-oxidant, tissue protecting, antithrombotic and mucolitic agents.

**Revendications**

1. Composés de formule I

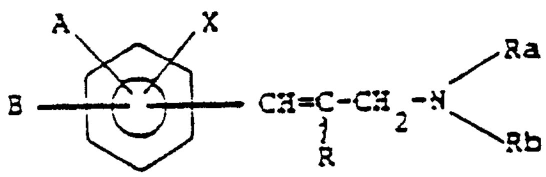

(I)

dans laquelle:

- X représente un groupe OH ou

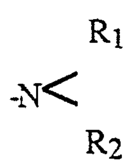

où $R_1$ et $R_2$, qui peuvent être identiques ou différents, représentent un groupe alkyle en $C_2$-$C_6$ à chaîne droite ou ramifiée, un groupe cycloalkyle en $C_3$-$C_6$, un groupe cyclopropylméthyle, benzyle, hydroxyéthyle, chloroéthyle, ou les groupes $R_1$ et $R_2$, forment, conjointement avec l'atome d'azote, un groupe pipérazine-1-yle, 4-N-acétyl-pipérazine-1-yle, N-méthyl-pipérazine-1-yle, ou un groupe aziridinyle;
- R est choisi dans le groupe formé par l'hydrogène, un groupe alkyle en $C_1$-$C_4$ à chaîne droite ou ramifiée, un groupe cyano ou un groupe carboxyle estérifié;
- A et B, qui peuvent être identiques ou différents, sont choisis dans le groupe formé par l'hydrogène, un groupe alkyle en $C_1$-$C_4$ à chaîne droite ou ramifiée, un groupe alcoxy en $C_1$-$C_2$, un atome d'halogène, un groupe 1-imidazolyle ou un groupe de formule

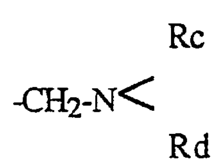

;

- Ra, Rb, Rc et Rd, qui peuvent être identiques ou différents, sont choisis dans le groupe formé par un groupe alkyle en $C_1$-$C_4$ à chaîne droite ou ramifiée, un groupe cycloalkyle en $C_3$-$C_6$, un groupe cyclopropylméthyle, hydroxyéthyle, chloroéthyle, les groupes de formules

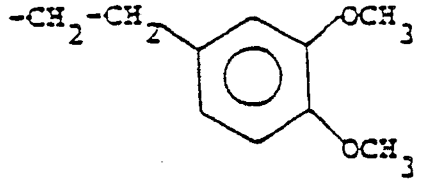
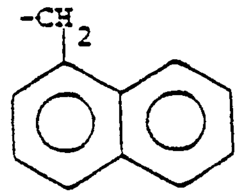
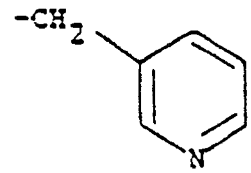

ou les substituants d'un groupe amino disubstitué, conjointement avec l'atome d'azote, représentent un noyau hétérocyclique azoté saturé ou insaturé, choisi dans le groupe formé par les groupes morpholine-4-yle, pyrrolidin-1-yle, pipéridine-1-yle, pipérazine-1-yle, N-méthyl-pipérazine-1-yle, N-p-fluorophényl-pipérazine-1-yle, N-(phénylthiométhyle)-pipérazine-1-yle, N-(bis-phényl-méthyl)pipérazine-1-yle, N-(bis-p-fluorophénylméthyl)-pipérazine-1-yle, aziridinyle, 2-carboxy-pyrrolidine-1-yle, 2-cyano-pyrrolidine-1-yle, 3-thiazolidinyle, 4-carboxy-3-thiazolidinyle;
- étant entendu que le substituant X et la chaîne propényle sont en position ortho ou para, et que les substituants A et B sont dans les autres positions libres ortho et para du cycle, comme représenté par les formules Ia et Ib:

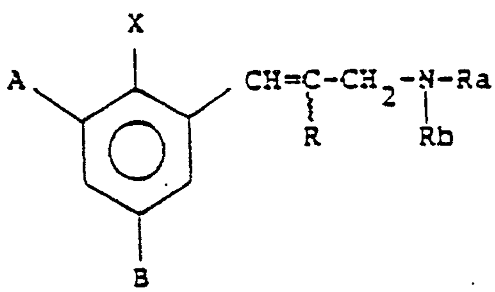
(Ia)

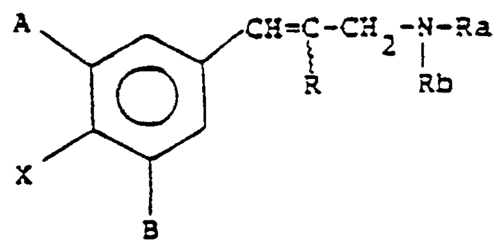
(Ib)

étant entendu que lorsque $-NR_aR_b$ représente le groupe N-(bis-p-fluorophénylméthyl)-pipérazine, A ou B ou les deux doivent représenter le groupe $-CH_2-NR_aR_b$,
leurs sels non toxiques, leurs isomères optiques et géométriques et les mélanges de ces composés.

**2.** Composés selon la revendication 1, dans lesquels X représente un groupe hydroxyle, A et B représentent un atome d'hydrogène, un groupe méthoxy, bromo, fluoro, ou un reste de formule

$$-CH_2-N{<}^{Ra}_{Rb} \quad ,$$

où Ra et Rb ont les mêmes significations que dans la revendication 1.

**3.** Composés selon la revendication 1, dans lesquels X représente un groupe de formule

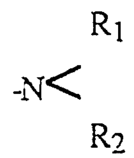

,

où $R_1$ et $R_2$ sont définis comme à la revendication 1, A et B représentent l'hydrogène et R est un groupe cyano, un atome d'hydrogène ou un groupe méthyle.

4. Procédé de préparation des composés selon les revendication 1 à 3, caractérisé en ce qu'un acide de formule II ou son sel de sodium ou de potassium

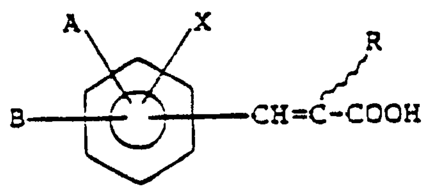

(II)

où A, B, X et R sont définis comme ci-dessus, est mis à réagir avec le formaldéhyde et une amine secondaire de formule III

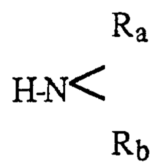

(III)

dans laquelle Ra et Rb sont définis comme ci-dessus.

5. Procédé selon la revendication 4, caractérisé en ce que la réaction est effectuée dans des solvants choisis dans le groupe formé par l'eau, les solutions aqueuses de carbonates ou de bicarbonates alcalins, les alcools inférieurs en $C_1$-$C_3$, le diméthoxyéthane, le tétrahydrofurane, le diglime et leurs mélanges.

6. Procédé selon la revendication 4 ou 5, caractérisé en ce que les proportions molaires de l'acide II et de l'amine III sont sensiblement équivalentes.

7. Procédé selon la revendication 4 ou 5, caractérisé en ce que par rapport à l'acide II, on utilise un excès de formaldéhyde ou d'amine qui peut être dans la gamme de 3:1 à 6:1.

8. Compositions pharmaceutiques contenant, comme principe actif, au moins un des composés des revendications 1 à 3 et un véhicule pharmaceutiquement acceptable.

9. Composés selon les revendications 1 à 3, utilisés comme agents thérapeutiques.

10. Composés selon les revendications 1 à 3, utilisés comme "scavengers" (balayeurs) de radicaux, agents antioxydants, protecteurs de tissus, agents antithrombotiques et agents mucolytiques.

**Ansprüche**

1. Verbindungen der Formel I

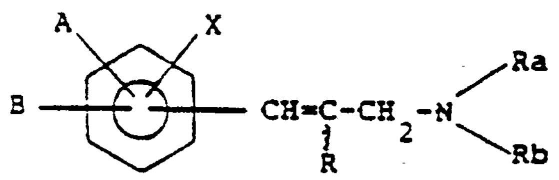

(I)

in welcher

- X OH oder

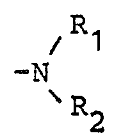

bedeutet, wobei $R_1$ und $R_2$, die gleich oder verschieden sind, geradkettiges oder verzweigtes $C_2$-$C_6$-Alkyl, $C_3$-$C_6$-Cycloalkyl, Cyclopropylmethyl, Benzyl, Hydroxymethyl, Chlorethyl oder $R_1$ und $R_2$ zusammen mit dem Stickstoffatom einen Piperazin-1-yl, 4-N-Acetyl-piperazinyl-1-yl, N-Methyl-piperazinyl-1-yl, N-Methyl-piperazinyl-1-yl oder Aziridinyl-Rest bilden,
- R ausgewählt wird aus der Gruppe Wasserstoff, $C_1$-$C_4$-geradkettiges oder verzweigtes Alkyl, Cyano oder verestertes Carboxyl,
- A und B, die gleich oder verschieden sind, ausgewählt werden aus der Gruppe Wasserstoff, $C_1$-$C_4$-geradkettiges oder verzweigtes Alkyl, $C_1$-$C_2$-Alkoxy, Halogen, 1-Imidazolyl oder der Formel

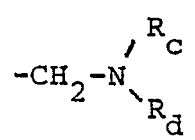

- $R_a$, $R_b$, $R_c$ und $R_d$, die gleich oder verschieden sind, ausgewählt werden aus der Gruppe $C_1$-$C_4$-geradkettiges oder verzweigtes Alkyl, $C_3$-$C_6$-Cycloalkyl, Cyclopropylmethyl, Hydroxyethyl, Chlorethyl, Gruppen der Formel

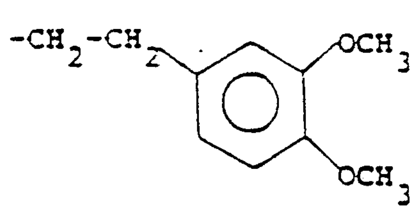

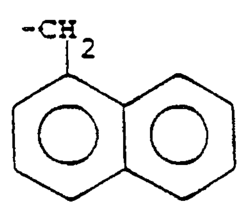

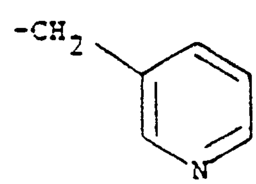

oder die Substituenten der disubstituierten Aminogruppe bilden zusammen mit dem Stickstoffatom einen gesättigten oder ungesättigten stickstoffhaltigen heterocyclischen Ring,der ausgewählt wird an der Gruppe gebildet durch Morpholin-4-yl, Pyrrolidin-1-yl, Piperidin-1-yl, Piperazin-1-yl, N-Methyl-piperazin-1-yl, N-p-Fluorphenyl-piperazin-1-yl, N-(Phenylthiomethyl)-piperazin-1-yl, N-(Bis-phenylmethyl)piperazin-1-yl, N-(Bis-p-fluoerphenylmethyl)-piperazin-1-yl)` Aziridinyl, 2-Carboxy-pyrrolidin-1-yl, 2-Cyano-pyrrolidin-1-yl, 3-Thiazolidinyl, 4-Carboxy-3-thiazolidinyl,
- mit der Bedingung, daß der Substituent X und die Propenylkette in ortho- oder para-Stellung stehen und dar die Substituenten A und B in den anderen freien ortho- oder para-Stellungen des Ringes stehen, wie dargestellt in den Formeln Ia und Ib

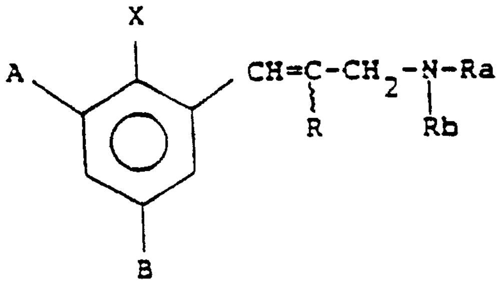

(Ia)

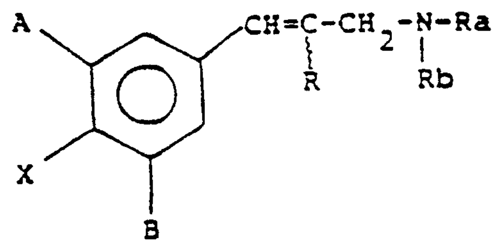

(Ib)

- mit der Maßgabe, daß wenn $-NR_aR_b$ N-(Bis-p-fluorphenylmethyl)-piperazinyl bedeutet, A oder B oder beide die Gruppe $-CH_2-NR_aR_b$ sein müssen,
ihre nicht toxischen Salze, optischen und geometrischen Isomere und Mischungen davon.

**2.** Verbindungen gemäß Anspruch 1, worin X eine Hydroxy-Gruppe, A und B Wasserstoff, Methoxy, Brom, Fluor oder einen Rest der Formel

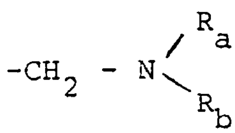

bedeuten, in der $R_a$ und $R_b$ die gleiche Bedeutung haben wie in Anspruch 1.

**3.** Verbindungen gemäß Anspruch 1, worin X eine Gruppe der Formel

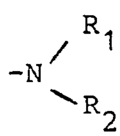 ,

in der $R_1$ und $R_2$ die in Anspruch 1 angegebene Bedeutung haben, A und B Wasserstoff und R Cyano, Wasserstoff oder Methyl bedeuten.

**4.** Verfahren zur Herstellung von Verbindungen gemäß Ansprüche 1 bis 3, dadurch gekennzeichnet, daß man eine Säure der Formel II oder ihr Natrium- oder Kalium-Salz

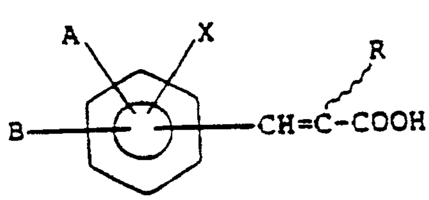

(II)

in der A, B, X und R die oben angegebenen Bedeutungen haben, mit Formaldehyd und einem sekundären Amin der Formel III

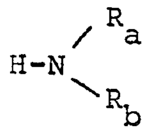

(III)

in der $R_a$ und $R_b$ die oben angegebenen Bedeutungen haben, umsetzt.

5. Verfahren gemäß Anspruch 4, dadurch gekennzeichnet, daß die Reaktion in Lösungsmitteln durchgeführt wird ausgewählt aus der Gruppe Wasser, wäßrige Alkali-carbonat- oder bicarbonat-Lösungen, niedere $C_1$-$C_3$-Alkohole, Dimethoxyethan, Tetrahydrofuran, Diglyme und Mischungen davon.

6. Verfahren gemäß Ansprüche 4 oder 5, dadurch gekennzeichnet, daß die molaren Verhältnisse zwischen Säure II und Amin III äquivalent sind.

7. Verfahren gemäß Ansprüche 4 oder 5, dadurch gekennzeichnet, daß ein Überschuß an Formaldehyd und Amin eingesetzt wird bezüglich der Säure II, der von 3 : 1 bis 6 : 1 betragen kann.

8. Pharmazeutische Zubereitungen, die als Wirkstoff mindestens eine der Verbindungen gemäß Ansprüche 1 bis 3 und einen pharmazeutisch verträglichen Träger enthalten.

9. Verbindungen gemäß Ansprüche 1 bis 3 zur Verwendung als Arzneimittel.

10. Verbindungen gemäß Ansprüche 1 bis 3 zur Verwendung als "Radikalfänger", Antioxidations-, Gewebeschutz-, antithrombotisches und mucolytisches Mittel.